# EUROPEAN PATENT SPECIFICATION

(11) **EP 1 978 979 B1**
(45) Date of publication and mention of the grant of the patent: **13.08.2014**
(21) Application number: 07702565.8
(22) Date of filing: 02.01.2007
(51) Int. Cl.: A61K 35/74, A61K 8/99, A61P 1/02

(54) **PROBIOTIC ORAL HEALTH PROMOTING PRODUCT**
PROBIOTISCHES ORALES GESUNDHEITSFÖRDERNDES PRODUKT
PRODUIT PROBIOTIQUE POUR L'AMÉLIORATION DE L'HYGIÈNE ORALE

(30) Priority: 04.01.2006 SE 0600030; 19.01.2006 US 759926 P
(43) Date of publication of application: 15.10.2008
(73) Proprietor: Wikström, Maude, SE-411 33 GÖTEBORG (SE); Almståhl, Annica, 446 35 Älvängen (SE)
(72) Inventor: Wikström, Maude, SE-411 33 GÖTEBORG (SE); Almståhl, Annica, 446 35 Älvängen (SE)
(74) Representative: Valea AB
(86) International application number: PCT/EP2007/000015
(87) International publication number: WO 2007/077210

(56) References cited:
- EP-A2- 0 195 672
- WO-A-96/40865
- WO-A1-2005/018342
- US-B1- 6 290 934
- DATABASE MEDLINE [Online] US NATIONAL LIBRARY OF MEDICINE (NLM), BETHESDA, MD, US; 1990, NYVAD B ET AL: "Comparison of the initial streptococcal microflora on dental enamel in caries-active and in caries-inactive individuals." XP002431478 Database accession no. NLM2276164 & CARIES RESEARCH 1990, vol. 24, no. 4, 1990, pages 267-272, ISSN: 0008-6568
- DATABASE WPI Week 200219, Derwent Publications Ltd., London, GB; AN 2002-140573 & CN 1 321 462 A (AI M) 14 November 2001

## Description

### TECHNICAL FIELD

The present invention relates to the re-establishment of an oral microflora associated with good oral health.

### BACKGROUND OF THE INVENTION

Under normal conditions, the composition of the oral microflora is balanced in such a way that health-related micro-organisms are dominating. Factors which can disturb the balance in the normal oral flora are for example hyposalivation (reduced salivary secretion), frequent intake of fermentable carbohydrates, neglected oral hygiene, antibiotic treatment and reduced host defense.

At hyposalivation, the pH and buffer capacities are often found reduced. This leads to an increase in acid-producing and acid-tolerant micro-organisms like mutans streptococci, *Lactobacillus* spp and *Candida* as well as generally tolerant micro-organism like staphylococci, enterics and enterococci. Also frequent intake of fermentable carbohydrates favors acid-tolerant and acid-producing micro-organisms. The number of *Lactobacillus* cells in the oral cavity is positively correlated to the intake of dietary carbohydrates. When oral hygiene is neglected, micro-organisms accumulate on the tooth surfaces forming dental plaque. The accumulation will, in a couple of weeks, result in a decrease in facultative anaerobic Gram-positive bacteria and an increase in anaerobic Gram-negative bacteria associated with gingival inflammation and development of periodontal disease. An increase in staphylococci, enterics, enterococci and coliforms might also be the result when oral hygiene is neglected. Antibiotic treatment often leads to an increase in *Candida,* staphylococci, enterococci and coliforms. It has been known for a long time from endodontic treatment that enterococci are resistant to most antibiotics and disinfectants and are very difficult to eliminate. Further, multiple-drug resistant staphylococci and, during the lasts years, also enterococci are important nosocomial pathogens. In subjects with an impaired host defense due to immunosuppressive chemotherapy, acute leukemia or terminal illness increases in *Candida,* staphylococci, enterococci, coliforms *Pseudomonas* and Gram-negative rods are regularly found. Studies also show that all subjects with hyposalivation due to radiation-therapy in the head and neck region, and most subjects with Sjögren's syndrome, harbor enterococci while these micro-organisms are not detected in matched healthy controls.

Of the above mentioned factors disturbing the normal healthy oral flora, hyposalivation is the factor most studied. About 25% of the adult population suffers from oral dryness, which is a symptom of hyposalivation. Common reasons for hyposalivation are radiation therapy in the head and neck region, rheumatic disorders, hormonal changes, depression, diabetes mellitus and drugs.

Drugs, which may have hyposalivation as a side effect, are for example anti-hypertensives, anti-depressants, diuretics, psychotropics and cytostatics. Also drugs which by themselves are not known to cause hyposalivation, can give this problem when 4-5 drugs or more are taken together. Multi-drug treatment is common among elderly and about 70% in the age group 65 years and older, experience discomfort related to hyposalivation.

In all groups, the most notable change in the oral microflora is an increase in the number and proportion of lactobacilli. Lactobacilli are known to ferment a wide range of carbohydrates, resulting in acid production. In the last years it has been shown that species of lactobacilli are able to ferment the often used sugar substitutes sorbitol and xylitol, with acid production as a result. Lactobacilli are also known to survive and multiply at very low pH. In subjects with good oral health and normal salivary secretion rate, the proportion of lactobacilli is, with few exceptions, below detectable levels in saliva samples when analyzed with cultivation techniques.

Products containing naturally occurring bacteria with host-beneficial effects, so-called probiotics, have been developed and successfully used for the treatment of disturbances in the intestinal flora. The probiotics most often used in these products are lactobacilli. Up to now a few studies are available on the effect of probiotics on the oral health, most of which also concern the use of lactobacilli. US2004/0101495, WO2004/067729, WO0009080, EP0524732, WO03017951, WO0078322 all disclose a use of lactic bacteria that are not part of the resident microflora of the mouth, as a method of treating or preventing dental caries, dental plaque, periodontal infections or halitosis. However, as discussed above, subjects with a disturbed oral microflora mostly have high proportions of lactobacilli and a high caries incidence. Thus, the use of lactobacilli as a probiotic for subjects with a disturbed oral microflora due to hyposalivation could be questioned.

With a few exceptions other micro-organisms than lactobacilli have been used as probiotics. WO 2005/007178 discloses a method in which BLIS-producing *Streptococcus salivarius* strains are used for inhibiting growth of anaerobic bacteria, particularly halitosis causing bacteria. The primary habitat for *S*. *salivarius* is the dorsum of the tongue. In subjects with a high sucrose-consumption *S*. *salivarius* can be detected in the supragingival plaque. When sucrose is available *S*. *salivarius* produce large amounts of easily soluble extra-cellular polysaccharides. These polysaccarides can be used both by themselves and by other cariogenic bacteria, like mutans streptococci, in periods of "sucrose-starvation" resulting in a prolonged acid production and low pH in the dental plaque.

WO 96/40865 discloses compositions comprising one or more lactate dehydrogenase (LDH)-deficient mutans streptococcus strains for prevention and/or treatment of dental caries in a dental caries-susceptible host. When live recombinant *S*. *mutans* cells are to be used it is suggested that the cells are stored in buffered saline solutions or culture media until intake to maintain their viability. The proposed function of the LDH-deficient mutans streptococci is to prevent colonization of the tooth surface by other pathogenic micro-organisms such as for example *S*. *mutans.* However, mutans streptococci do not belong to the early colonisers of a clean tooth surface, they are mainly found in subjects with high sugar-consumption and high caries-activity, and they do not belong to the healthy normal oral flora. WO2005/018342 discloses compositions comprising one or more LDH-deficient *mutans streptococcus* strains together with one or more *Streptococcus uberis* strains and/or one or more *Streptococcus oralis* strains. *S*. *uberis* has the potential to interfere with the colonization of periodontal pathogens. The use of *S*. *oralis* is based on their production of hydrogen peroxide and the ability of hydrogen peroxide to inhibit the growth of periodontal microbial pathogens. However, the peridontitis sites are characterised by a great number of host PMN-cells (polymorphnuclear leukocytes) with a considerable capacity to release hydrogen peroxide, why the additional hydrogen peroxide production by *S*. *oralis* might have marginal effect.

The carbonic acid/bicarbonate system is the most important buffer system in saliva for neutralising the acids produced by micro-organisms. The main part of bicarbonate in saliva is that released from the salivary glands at chewing. A decreased buffer capacity leads to prolonged periods of low pH after meals, which favours acid-tolerant micro-organisms. Generally the pH of the saliva in subjects with a disturbed oral microflora is low compared to subjects with healthy oral conditions.

To our knowledge, there are no probiotic products available for subjects having a disturbed microflora for other reasons than bad breath and gingival inflammation.

There are several products available on the market for subjects suffering from oral dryness. The majority of these products contain sorbitol and/or xylitol as sweeteners. However, several *Lactobacillus, Enterococcus* and *Candida* species are able to use these sweeteners as nutrients and to produce acids from them. Most products are intended to stimulate the salivary glands. For this purpose acid compounds, for example malic acid or citric acid, are used. The use of acid compounds might however further decrease the oral pH. It should be noted that saliva stimulating products are not effective for subjects with severe oral dryness. For subjects with almost no measurable salivary secretion there are gels, saliva-substitutes and products with lubricating and/or moisturising effect. These products have no documented effect on the oral pH or on the microbial flora.

Bicarbonate is used in the US patent application 2004/0115138 which discloses a dentifrice gel or paste comprising baking soda, NaHCO₃ (40-70 % by weight) in combination with other ingredients such as flavouring oils, xanthan gum binder, surfactants, humectants and some natural ingredients as an oral health care formulation.

US 4,618,489 uses bicarbonate in a concentration of 10-120 mM together with 20-100 mM of other ions such as for example fluoride, chloride or thiocyanate. In this invention bicarbonate is used for its ability to raise the oral pH and thereby enhance the activity of the enzymes lysozyme, trypsin and chymotrypsin and decrease the number of *S*. *mutans, Lactobacillus casei* and *Actinomyces* species in the mouth. Lysozyme is a constituent of the saliva with the ability to degrade cell-wall protein components of Gram-positive bacteria. The majority of the bacteria in the normal healthy mouth are Gram-positive. The trypsin and chymotrypsin found in the oral cavity is that mainly released from host PMN cells at gingival inflammation, periodontal disease and other mucosal inflammatory sites. One of the characteristics of an inflammatory site is a pH above normal due to pH rising products in serum.

Carbamide is also a pH rising component naturally present in the saliva. It is commonly used in teeth whitening compositions and in chewing gums but in US 6,290,934 carbamide perhydrate is the active ingredient in an agent to promote oral hygiene and health, such as the inhibition of bacterial plaque and caries prophylaxis. However, none of the pH-rising or buffer substances are used in combination to promote the growth of the probiotic bacteria in the oral cavity.

It is well known that microbial species associated with caries and mucosal infections are favoured by an acid pH, and that microbial species associated with the development of periodontal disease are favoured by a pH above normal, and microbial species associated with good oral health by a neutral pH. It is also well known that bicarbonate is one of the principal buffering components in saliva. Also, it is generally agreed that the microbial degradation of carbamides (urea), present in saliva and gingival exudates and incorporated into the dental biofilm, contribute to keep the oral pH at a "healthy" level. Our research has shown that subjects with hyposalivation have a changed microflora with an increase in acidogenic, aciduric and acidtolerant microbial species and a decrease in streptococcal species associated with oral health. The results indicate that the use of a bacterial strain of an early colonizing species, which is naturally occurring in high numbers at healthy oral conditions, in combination with naturally occurring pH-rising components to support its colonisation, will promote the return to a normal healthy oral flora especially in subjects with hyposalivation.

The present invention will provide a combination of probiotic bacteria and pH-rising components, which re-establishes an oral microflora associated with good oral health in subjects with a disturbed oral microflora. The invention can be added to different kinds of oral health promoting products.

### SUMMARY OF THE INVENTION

The present invention discloses an oral composition according to claim 1.

One further aspect of this invention is to provide such an oral composition for use in the re-establishment of good oral health.

One further aspect of this invention is to provide the use of such an oral composition for the re-establishment of good oral health in subjects suffering from oral dryness.

In one preferred embodiment of the invention, the weak or non acid-producing bacterial strain is *Streptococcus oralis.*

In one preferred embodiment of the invention the weak or non acid-producing bacterial strain is *Streptococcus vestibularis.*

In one preferred embodiment of the invention the weak or non acid-producing bacterial strain is *Eubacterium saburreum or Eubacterium yurii*

In one preferred embodiment of the invention the weak or non acid-producing bacterial strain is *Neisseria mucosa* or *Neisseria subflava*

In one preferred embodiment of the invention the weak or non acid-producing bacterial strain is *Veillonella parvula* or *Veillonella dispar.*

In one preferred embodiment of the invention the weak or non acid-producing bacterial strains are genetically modified.

In one preferred embodiment of the invention the pH-rising and/or pH-buffering substance is one, or a combination of two or more of the substances chosen from the group comprising bicarbonates, carbamides, phosphates, proteins and/or salts.

In one preferred embodiment of the invention the pH-rising and/or pH-buffering substance is sodium bicarbonate, NaHCO₃ and/or carbamide or a combination of these.

In one preferred embodiment of the invention the pH-rising and/or pH-buffering substances are used in a concentration ranging from 15-300 mmol/l oral composition, more preferably 30-200 mmol/l oral composition and most preferably 50-100 mmol/l oral composition.

In one preferred embodiment of the invention the bacteria are freeze dried.

In one preferred embodiment of the invention the freeze dried or living bacteria are immersed in oil.

In one preferred embodiment'of the invention a lubricant has been added chosen from the group comprising edible oils, essential oils, glycerin, carboxymethyl cellulose, xanthan gum or animal mucin.

In one preferred embodiment of the invention the lubricant is sun flower oil.

In one preferred embodiment of the invention a fluoro compound from the group comprising sodium fluoride, monofluorophosphate or stannous fluoride has been added.

In one preferred embodiment of the invention zinc and/or chlorine dioxide has been added.

In one preferred embodiment of the invention an anti-inflammatory substance from the group comprising cortison, benzydamin, non-steroid anti-inflammatory drugs or herbal extracts such as for example calendula extract or tee tree oil, have been added.

In one preffered embodiment of the invention an analgesic from the group comprising lidocaine or prilocaine has been added.

In one preferred embodiment of the invention an anti-mycotic agent from the group comprising amfotericin, flucanozol or nystatin has been added.

In one preferred embodiment of the invention one or more flavouring substances from the group comprising mints, fruit juices, liquorice, Stevia rebaudiana, steviosides, rebaudioside A, essential oils like eucalyptus oil, menthol has been added.

In one preferred embodiment of the present invention the oral composition is used for the re-establishment of good oral health in subjects suffering from oral dryness.

### DETAILED DESCRIPTION OF THE INVENTION

Micro-organisms associated with good oral health are favoured by a pH above pH 7. The expression "good oral health" is intended to have the following meaning; no active carries lesions, no or only a few sites with gingival inflammation and no signs or symptoms of oral mucosal infections. To enable probiotic bacteria to establish in the oral cavity in subjects with a disturbed oral microflora the pH needs to be increased.

By a combination of probiotic bacteria and pH-rising components, for example bicarbonate and/or carbamide, the proportion of bacteria associated with oral health will increase and the proportion of acid-producing and acid-tolerant micro-organisms decreases.

The invention can be used in daily home-care situations as well as in the dental practice and in assisted dental care. The invention is intended for subjects with a disturbed oral microflora, but will also be safe to use for all kinds of subjects in all age groups.

In the following the invention will be described in more detail. However, the described embodiments mentioned below are only given as examples and should not be limiting to the present invention. Other solutions, uses, objectives, and functions within the scope of the invention as claimed in the below described patent claims should be apparent for the person skilled in the art. It should also be noted that the word "comprising" does not exclude the presence of other elements or steps than those listed, and the words "a" or "an" preceding an element do not exclude the presence of a plurality of such elements.

### Saliva in patients suffering from oral dryness.

The salivary secretion rates, pH, buffer capacity and concentrations of bicarbonate and proteins in stimulated saliva have been measured in subjects with oral dryness of various origins. The results are presented in Table 1.

**Table 1. Unstimulated and stimulated secretion rate (ml/min), pH and buffer capacity in stimulated saliva, and concentrations of bicarbonate and saliva proteins in stimulated whole saliva in subjects with oral dryness due to medicines, primary Sjögrens syndrome or radiation therapy in the head and neck region and in controls with normal salivary secretion rate. Mean±sd (median value) are presented. Statistically significant differences compared with the controls are denoted with *.**

| | Medicines | Primary Sjögren's Syndrome | Radiation therapy | Controls |
|---|---|---|---|---|
| Unstimulated secretion rate | 0.04±0.04* (0.03) | 0.01±0.02* (0.01) | 0.02±0.03* (0.0) | 0.31±0.15 (0.30) |
| Stimulated secretion rate | 0.93±0.54* (0.90) | 0.47±0.38* (0.41) | 0.35±0.38* (0.26) | 2.0±0.76 (1.9) |
| pH | 7.5±0.3 (7.5) | 7.2±07* (7.4) | 6.5±1.1* (6.7) | 7.6±0.2 (7.5) |
| Buffer capacity | 5.6±1.1* (6.0) | 5.2±1.2*, (5.6) | 4.8±1.2* (4.9) | 5.9±0.8 (6.2) |
| Bicarbonate (mM) | 10±4* (8) | 7±5* (7) | 7±5* (7) | 14±3.5 (14) |
| Total protein (mg/ml) | 1.0±0.3 (0.9) | 1.8±1.4* (1.4) | 2.5±1.9* (1.8) | 0.9±0.2 (0.9) |
| Albumin (mg/ml) | 0.3±0.2 (0.2) | 1.3±1.1* (1.0) | 1.3±0.8* (1.1) | 0.2±0.1 (0.2) |
| Lactoferrin (µg/ml) | 5.0±3.6 (4.3) | 8.8±9.0* (5.8) | 72±48* (65) | 3.7±2.5 (2.7) |
| Amylase (kUnits/ml) | 3.1±1.8 (2.9) | 2.6±1.4 (2.3) | 1.4±1.2* (1.1) | 3.3±1.7 (3.2) |
| Mucin (Units/ml) | 2.5±2.7 (1.5) | 3.0±3.2 (2.2) | 1.7±0.8 (1.8) | 2.4±1.7 (2.1) |

The unstimulated secretion rate is almost absent in most subjects with moderate to severe oral dryness. The stimulated secretion rate is decreased with 50-80%. The pH in stimulated saliva is significantly decreased for the subjects with oral dryness due to radiation therapy in the head and neck region. For the groups with oral dryness due to medicines or due to primary Sjögren's syndrome, the pH is close to normal in stimulated saliva but most likely lower in the unstimulated state. The buffer capacity and the concentration of bicarbonate are significantly lower for all three groups with oral dryness compared with the controls. The concentrations of lactoferrin and albumin in saliva are significantly higher in the primary Sjögren's syndrome group and the radiation therapy groups indicating inflammation and leakage of serum through fragile mucosal membranes. In subjects with a high caries activity as well as in subjects with a disturbed oral microflora for other reasons, a low pH and a decreased buffer capacity is common despite normal salivary secretion rates.

### Oral microflora in subjects with hyposalivation

The oral microflora on the tongue, buccal mucosa, vestibulum in the molar region, in supragingival plaque and in the subgingival region have been analyzed in subjects with hyposalivation due to medicines, primary Sjögren's syndrome or radiation therapy in the head and neck region and in controls with normal salivary secretion rates. The results are presented in Tables 2-6.

**Table 2. Total count and numbers of specific micro-organisms expressed as log 10 on the tongue. Mean±sd (median value) are presented. Statistically significant differences compared with controls are denoted with *.**

| | Medicines | Primary Sjögrens Syndrome | Radiation therapy | Controls |
|---|---|---|---|---|
| Total count | 6.8±0.4 (6.8) | 7.1±0.8 (7.4) | 5.8±0.7* (5.9) | 6.8±0.7 (6.8) |
| Streptococci | 6.6±0.5* (6.2) | 6.9±0.6* (7.0) | 5.3±0.6* (5.4) | 6.2±0.6 (6.2) |
| *S. salivarius* | 5.2±1.4 (5.5) | 6.2±0.7* (6.3) | 3.6±2.2* (4.5) | 5.2±1.2 (5.5) |
| *F. nucleatum* | 3.8±1.3 (4.1) | 3.1±1.8 (3.0) | 0.7±1.1* (0.0) | 3.7±1.8 (4.3) |
| *P. intermedia* | 1.5±1.4 (1.7) | 2.4±1.8 (2.9) | 0.5±1.3* (0.0) | 11.7±1.9 (1.7) |
| *Staph. aureus* | 0.2±0.5 (0.0) | 0.3±0.8 (0.0) | 0.9±1.5 (0.0) | 0.3±0.7 (0.0) |
| *C. albicans* | 0.3±0.7 (0.0) | 0.3±0.7 (0.0) | 0.8±1.1* (0.0) | 0.0±0.0 (0.0) |
| Enterococci | 0.3±0.8 (0.0) | 1.4±1.6* (0.0) | 2.5±1.6* (2.9) | 0.1±0.5 (0.0) |
| Enterics | 0.0±0.0 (0.0) | 0.4±1.0 (0.0) | 0.4±0.9 (0.0) | 0.1±0.4 (0.0) |

**Table 3. Total count and numbers of specific micro-organisms expressed as log 10 on the buccal mucosa. Meanisd (median value) are presented. Statistically significant differences compared with controls are denoted with ***

| | Medicines | Primary Sjögrens Syndrome | Radiation therapy | Controls |
|---|---|---|---|---|
| Total count | 5.4±0.6 (5.3) | 5.6±0.5* (5.6) | 5.3±0.5 (5.4) | 5.1±0.7 (5.3) |
| Streptococci | 5.1±0.6 (5.1) | 5.4±0.5* (5.4) | 5.0±0.6 (5.0) | 4.9±0.6 (4.8) |
| *S*. *sanguis*/*oralis* | 3.3±1.6 (3.6) | 3.4±1.3 (3.7) | 2.4±1.6* (3.0) | 3.5±1.0 (3.7) |
| *P. intermedia* | 1.5±1.4 (1.7) | 1.1±1.4 (0.0) | 0.6±1.2* (0.0) | 1.6±1.3 (1.7) |
| *Staph. aureus* | 0.2±0.6 (0.0) | 0.4±0.8 (0.0) | 0.8±1.0 (0.0) | 0.1±0.4 (0.0) |
| *C*. *albicans* | 0.1±0.6 (0.0) | 0.2±0.6 (0.0) | 0.2±0.6* (0.0) | 0.1±0.4 (0.0) |

**Table 4. Total count and numbers of specific micro-organisms expressed as log 10 in the vestibulum in the molar region. Mean±sd (median value) are presented. Statistically significant differences compared with controls are denoted with *.**

| | Medicines | Primary Sjögren's Syndrome | Radiation Therapy | Controls |
|---|---|---|---|---|
| Total count | 5.9±0.7* (5.8) | 5.6±0.7 (5.6) | 5.9±0.7* (6.0) | 5.4±0.8 (5.5) |
| Streptococci | 5.6±0.8 (5.4) | 5.5±0.8 (5.5) | 5.5±0.7 (5.5) | 5.1±0.9 (5.4) |
| *F. nucleatum* | 2.2±1.3 (2.4) | 2.3±1.2 (2.5) | 1.3±1.3 (1.7) | 2.0±1.2 (2.3) |
| *P. intermedia* | 1.5±1.8 (0.0) | 1.4±1.4 (1.9) | 0.7±1.6 (0.0) | 1.1±1.3 (0.0) |
| *Staph. aureus* | 0.4±1.0 (0.0) | 0.3±0.8 (0.0) | 0.2±0.6 (0.0) | 0.0±0.0 (0.0) |
| *C. albicans* | 0.1±0.5 (0.0) | 0.3±0.8 (0.0) | 1.3±1.4 (1.1) | 0.0±0.0 (0.0) |
| Enterococci | 0.3±0.9* (0.0) | 0.1±0.4* (0.0) | 2.5±1.1* (25) | 0.0±0.0 (0.0) |
| Enterics | 1.2±1.0 (0.0) | 0.5±1.0 (0.0) | 0.0±0.0 (0.0) | 0.0±0.0 (0.0) |

To summarize the results; in the radiation therapy group, the numbers of the acid-sensitive *S*. *salivarius*, *S*. *sanguis*/*S*. *oralis* and *Prebotella intermedia* are signficantly lower compared with the controls. The radiation therapy group also shows an increase in *C*. *albicans* and enterococci, associated with an acidic environment and mucosal infections. It should be noted that all subjects in the radiation therapy group harbor enterococci. In the primary Sjögren's syndrome group and in the medicines group, the number of enterococci is increased and the number of *C*. *albicans* tends to be increased. In subjects with an impaired host defense due to immunosuppressive chemotherapy, acute leukemia or terminal illness, increases in *Candida,* coliforms, enterococci, *Pseudomonas,* staphylococci and Gram-negative rods are found.

**Table 5. Total count and numbers of specific micro-organisms expressed as log 10 in supragingival plaque. Mean±sd (median value) are presented. Statistically significant differences compared with controls are denoted with *:**

| | Medicines | Primary Sjögren's Syndrome | Radiation therapy | Controls |
|---|---|---|---|---|
| Total count | 6.7±0.6* (6.6) | 6.5±0.5 (6.5) | 6.6±0.6 (6.5) | 6.1±0.9 (6.4) |
| Streptococci | 6.0±0.6* (6.0) | 5.9±0.5 (6.0) | 5.8±0.6 (5.7) | 5.5±0.8 (5.6) |
| Mutans strept. | 4.2±1.1* (4.1) | 4.4±1.4* (4.4) | 3.4±2.2 (3.8) | 2.3±1.8 (2.2) |
| Lactobacilli | 1.9±2.1* (1.7) | 2.6±2.2* (2.9) | 4.7±1.6* (5.0) | 0.2+0.7 (0.0) |
| *C. albicans* | 1.3±1.3* (1.7) | 1.6±1.5* (1.7) | 1.8±2.1* (0.0) | 0.5±1.1 (0.0) |

In the supragingival plaque, the number of mutans streptococci, strongly associated with caries are significantly higher in the medicines group and in the primary Sjögren's syndrome group and tends to be higher in the radiation therapy group. An increase in the number of mutans streptococci is also seen in subjects with a high caries activity. The number of lactobacilli, associated with caries and an acidic environment, is significantly higher in all three groups with oral dryness compared with the controls. An increase in lactobacilli is also seen in subjects with frequent intakes of easily fermentable carbohydrates. The number of *C*. *albicans* is also significantly increased in all three groups with oral dryness. An increase in *C*. *albicans* is a common side-effect of antibiotic treatment.

**Table 6. Total count and numbers of specific micro-organisms expressed as log 10 in the gingival crevice region. Mean±sd (median value) are presented. Statistically significant differences compared with controls are denoted with*.**

| | Medicines | Primary Sjögren's Syndrome | Radiation therapy | Controls |
|---|---|---|---|---|
| Total count | 5.8±0.5 (5.9) | 5.6±0.6 (5.8) | 5.8±0.5 (5.9) | 5.5±0.7 (5.5) |
| Streptococci | 4.9±0.8 (5.0) | 4.9±0.6 (4.9) | 5.2±0.8 (5.1) | 4.8±0.6 (5.0) |
| *F. nucleatum* | 2.3±1.4 (2.5) | 2.8±2.0 (2.7) | 1.5±1.6 (1.0) | 2.3±1.4 (2.4) |
| *P. intermedia* | 2.2±2.2 (2.3) | 2.6±1.9 (3.2) | 0.0±0.0 (0.0) | 2.4±1.8 (2.6) |

In the gingival crevice region, there are no statistically significant differences between subjects with oral dryness and controls. It should be noted that *Porhyromonas gingivalis,* acid-sensitive and associated with periodontal disease, was not detected in any subject. *Actinobacillus actinomyctemcomitans,* also associated with periodontal disease, was only detected in a few subjects and in very low numbers. A neglected oral hygiene leads to an increase in Gram-negative micro-organisms such as *F*. *nucleatum, P. intermedia* and *P*. *gingivalis..*

### Lactobacilli and fermentation of carbohydrates and suctar-substitutes

Sorbitol and xylitol are the most frequently used sweeteners in toothpastes, chewing-gums, saliva-stimulating tablets and fluoride-gels and rinses. Subjects with oral dryness daily use several of these products. Lactobacilli isolated from dental plaque of subjects with oral dryness due to primary Sjögren's syndrome or radiation therapy in the head and neck region have been examined. The ability of 58 strains of lactobacilli to ferment the carbohydrates glucose, fructose and sucrose and the sugar-substitutes mannitol, sorbitol and xylitol has been examined. It should be noted that 69 % of the lactobacilli strains fermented sorbitol and 24% fermented xylitol to a pH level below pH 5.5, which is critical for enamel demineralization. The results from the fermentation of carbohydrates and sugar-substitutes are presented in Table 7.

**Table 7. pH after fermentation of carbohydrates and sugar-substitutes among lactobacilli isolated from supragingival plaque of subjects with oral dryness due to primary Sjögren's syndrome or radiation therapy. Proportions (%) of the strains are presented.**

| Substance | pH | | | | | |
|---|---|---|---|---|---|---|
| | < 4.0 | > 4.0-< 4.5 | > 4.5-< 5.0 | > 5.0-< 5.5 | > 5.5-< 6.0 | > 6.0 |
| Glucose | 21 | 67 | 7 | 5 | 0 | 0 |
| Fructose | 26 | 61 | 7 | 5 | 0 | 0 |
| Sucrose | 14 | 30 | 33 | 9 | 9 | 2 |
| Mannitol | 0 | 0 | 66 | 17 | 2 | 16 |
| Sorbitol | 2 | 0 | 41 | 26 | 14 | 17 |
| Xylitol | 0 | 0 | 0 | 24 | 40 | 36 |

### Probiotic strains favourable to subjects with a disturbed oral microflora

To be optimal for use as a probiotic in the oral cavity, the bacteria should be a weak or non acid-producer and unable to ferment sugar-substitutes or only producing low amounts of acids when fermenting sugar-substitutes. By the expression "weak or non acid-producer" we mean bacteria that are only able to produce small amounts of acids, which leads to minor pH- changes, or bacteria not producing acids from carbohydrates. Ability to adhere to buccal epithelial cells and to the tooth surface is also a necessity. At healthy conditions, alfa-hemolytic facultatively anaerobic streptococci dominate in the oral cavity. Streptococcal species comprising *S*. *oralis, S*. *sanguis and S*. *mitis* are among the first species colonizing the oral cavity of newborn babies and is part of the normal oral microflora both on the mucosal membranes and in the early dental plaque. *S*. *oralis* is favoured by a neutral pH and is a weak acid-producer. S. *oralis* is not known to be involved in the development of oral diseases. Other streptococcal species associated with oral health and with potential to be probiotic is e.g. *Streptococcus vestibularis. S. vestibularis* is able to degrade carbamide leading to the release of ammonia and thereby an increased pH. Streptococcal species not suitable as oral probiotics are anaerobic streptococci, beta-hemolytic streptococci and *S*. *mutans.* Anaerobic streptococci and beta-hemolytic streptococci are associated with infectious diseases. *S*. *mutans* is not part of the normal healthy oral microflora. *S*. *mutans* needs a tooth surface to be able to colonize the oral cavity and is strongly associated with the development of caries.

Other genera of oral bacteria associated with healthy conditions, and possible probiotic candidates, are *Eubacterium, Neisseria* and *Veillonella. Eubacterium* is a heterogenous group of micro-organisms consisting of for example *E*. *saburreum* and *E*. *yurii.* Many species belonging to *Eubacterium* are difficult to cultivate, which have lead to a limited knowledge about their prevalence and proportion in the oral cavity. The DNA technique has made it possible to increase the knowledge about oral *Eubacterium.* Some species belonging to the genus *Neisseria,* such as for example *N. mucosa* and *N. subflava* are found on healthy oral mucosa and in the early dental plaque and are not associated with disease. The genus *Veillonella* comprise of species such as for example *V. parvula* and *V. dispar. V. parvula* has been found in early dental plaque and on buccal mucosa in healthy subjects. A characteristic feature for *Veillonella* species are that the do not ferment carbohydrates. Instead they can use lactic acid as an energy source resulting in acetic acid, which is a weaker acid than lactic acid. The use of genetically modified bacterial strains in our invention is also contemplated. Positive characteristics for a probiotic strain are inability to store intra- and extracellular polysaccharides, inability to use sugar-substitutes as energy source, low acid-production and ability to adhere to saliva-coated tooth surfaces and epithelial cells.

Strains used in the present invention are isolated from subjects with good oral health. The use of strains from typestrain collections may lead to conflict of interest. For sampling the buccal mucosa a sterile cotton-stick is used and for sampling tooth surfaces sterile tooth-picks are used. The cotton-sticks or tooth-picks are transferred both to bottles with transport media VMGA III and to eppendorf vials. For cultivation, selective and non-selective agar plates as well as liquid media are used. The bacterial strains are identified using colony morphology, biochemical tests and genetic characterization in accordance with established methods. For storage, the bacterial strains are cultivated on blood agar for 48 hours and are transferred to Cryobank tubes (stored at -70°C) and they are also freeze-dried. The strains are identified and stored at the Culture Collection University of Göteborg (CCUG). The ability of the probiotic bacterial strains to use different sugars and sugar-substitutes and their pH-lowering capacity have been examined. Strains of *Streptococcus, Neisseria, Eubacterium* and *Veillonella* with weak acid-producing ability or inability to utilise or produce acids from carbohydrates have been identified. For sugar-substitutes, some strains of Streptococcus were able to use sugar-substitutes but the effect on the pH was very small. Strains of *Neisseria, Eubacterium* and *Veillonella* were unable to use sugar-substitutes as an energy source.

Further, the abilities of the strains to adhere to saliva-coated buccal epithelial cells and to saliva-coated hydroxyapatite (the main component of enamel) beads have been tested in vitro. Briefly, strains were grown to late exponential phase. Five mg of hydroxyapatite beads for buccal epithelial cells were covered with 70 µl of clarified saliva from healthy subjects. Saliva coated beads or buccal epithelial cells were kept over night at 4°C and then washed with distilled water and HEPES buffer. Thereafter, they were inoculated with 100 µl of bacterial suspension (bacteria which had grown in medium supplemented with 10 µCi/ml ¹⁴C acetic acid). Adhesion took place during 45 min at 37°C, then unbound bacteria were washed away and the number of attached cells was determined using scintillation counting. The streptococcal species and *Neisseria* species showed the best ability to adhere to buccal epithelial cells and to hydroxyapatite. *Veillonella* species and *Eubacterium* species were also able to adhere to both epithelial cells and hydroxyapatite but not to the same extent.

### pH-rising components

By the expression "pH-rising component" is meant a component that leads to an increase in the pH and by the expression "buffer substance" is meant a component that by its presence in solution resists changes in pH when small quantities of an acid or an alkali are added to it.

As discussed above, oral probiotic bacterial strains will have difficulties to establish in the oral cavity unless the pH is above pH 7. Bicarbonate is naturally occurring in saliva and is the most important buffer component. Carbamide is another pH-rising component naturally present in saliva. The optimal pH in the oral cavity shall be above pH 5.5 up to pH 7.8, more preferably above pH 6 up to pH 7.5, most preferably above pH 6.5 up to pH 7.2. The pH-rising and/or pH-buffer substance used in this invention should be able to maintain the pH in the oral cavity within the preferred ranges. In subjects with normal salivary secretion rate the concentration of bicarbonate in stimulated saliva is 10-60 mmol/l. This concentration of bicarbonate is enough to neutralise the pH from about pH 4.5-5.5 up to about pH 7 after the ingestion of easily fermentable carbohydrates after 5-20 minutes. The effect of bicarbonate on plaque pH after sucrose-exposure has been tested in vitro. After a 1-minute exposure to a high-bicarbonate dentifrice, diluted to give a concentration of 1 mol/l, the pH rose rapidly to the starting pH of about 6.7 and showed no tendency to decline toward the critical pH over the 2-hour period of the experiment. The growth of the probiotic at different concentrations of bicarbonate and/or carbamide has been tested. The results showed that the growth of the probiotic bacteria was only slightly affected at the concentrations of bicarbonate and/or carbamide, which will be used in our invention. Preferred concentration ranges for bicarbonate and/or carbamide are 15-300 mmol/l oral composition, more preferably 30-200 mmol/l oral composition and most preferably 50-100 mmol/l oral composition. Other pH rising components, which will be considered for use in our invention are for example phosphates like di-potassium hydrogen orthophosphate, potassium di-hydrogen orthophosphate or disodium hydrogen phosphate. Proteins, which are naturally present in saliva, can also act as buffer substances such as glycoproteins, histatins and statherins.

### The present invention in combination with other products

Optionally, the invention is added to other active components in order to achieve desired therapeutic effects.

One such desired effect can be lubrication of the oral cavity. This can be achieved by the addition of some edible oil such as Olive oil in Extra Virgin, Virgin and other cold-pressed forms, Rapeseed oil which is prepared conventionally or cold-pressed, sunflower oil, soy oil, maize oil, cotton-seed oil, peanut oil, sesame oil, cereal germ oil such as wheat germ oil, grape kernel oil, palm oil,and palm kernel oil, linseed oil. The bacterial cells, fresh or freeze dried, with a concentration of 10⁸-10¹² cells/ml, together with a pH-rising or buffering component, such as for example bicarbonate and/or carbamide, which adjusts and maintains optimum pH, is added to the lubricant. To homogenise the mixture a detergent might be added. Other lubricating agents, which are contemplated to be used together with our invention, are for example essential oils such as for example eucalyptus oil, glycerin, carboxymethylcellulosa, xanthan gum or animal mucin.

Fluorides are well known for their caries-preventing effect. This effect is mainly due to their ability to strengthen the tooth surfaces. The critical pH for enamel demineralisation is lower in the presence of fluoride compared with the absence of fluoride. The composition of the present invention can optionally be combined with fluorides such as sodium fluoride, monofluorophosphate or stannous fluoride.

Stevia is a natural sweetener with zero calories, zero carbohydrates and a zero glycemic index. Harvested from a plant in the daisy family, stevia provides a healthy alternative to sugar or chemical sweeteners. The component of stevia extract that gives it its sweetness is a mixture of various steviol glycosides. The components of sweetness in stevia leaves are stevioside, rebaudioside A, C, D, E and dulcoside A.

The ability of stevia to inhibit the growth and reproduction of bacteria and other infectious organisms is important in restoring a natural oral microflora. Research shows that *Streptococcus mutans, Pseudomonas aeruginosa, Proteus vulgaris* and other microbes do not thrive in the presence of the non-nutritive stevia constituents. Stevia has been shown to lower the incidence of dental caries. This fact, combined with the naturally sweet flavor of the herb, makes it a suitable ingredient in a composition for the use in the re-establishment of good oral health.

Bad breath is usually caused by bacteria producing volatile sulphuric compounds in their metabolism of amino acids in the mouth. Bacteria such as *Treponema denticola, P. gingivalis, P. intermedia* and *Tannerella forsythensis* are able to produce volatile sulphur compounds. Neglected oral hygiene and periodontal disease are examples of conditions when bad breath is common. Zinc reduces the formation of sulphur compounds and chlorine dioxide rapidly reacts with sulphur gasses. To reduce bad breath, the composition of the present invention can optionally be combined with zinc and/or chlorine dioxide.

Saliva coats all the surfaces in the oral cavity. Hyposalivation often leads to inflamed oral mucosal membranes due to a reduced protection by saliva. Severe mucosal inflammation is also a well-known side-effect of radiation therapy to the head- and neck region. To provide an anti-inflammatory effect, the composition of the present invention can optionally be combined with anti-inflammatory agents such as substances like cortison, benzydamin, non-steroid anti-inflammatory drugs or herbal extracts such as for example calendula extract or tee tree oil.

Besides from radiation therapy to the head and neck region, severe mucosal inflammation can also occur during cytostatic therapy. Severe mucosal inflammation is often very painful. The composition of the present invention can optionally be combined with an analgesic such as for example lidocaine or prilocaine.

Antibiotic treatment and reduced host defence often leads to fungal infections. To reestablish the oral microflora associated with oral health the present invention can optionally be combined with anti-mycotic agents such as amfotericin, flucanozol or nystatin.

To give the oral composition a pleasant taste, flavouring substances such as for example mints, fruit juices, liquorice, Stevia rebaudiana, steviosides, rebaudioside A, essential oils like eucalyptus oil, or menthol can optionally be combined with the invention.

### Pharmaceutical formulations

The compounds of the present invention may be isolated in any level of purity by standard methods and purification can be achieved by conventional means known to those skilled in the art, such as distillation, recrystallization and chromatography.

Bacteria in probiotic products are live cells or freeze-dried cells. Live cells are used in products like yoghurt while freeze-dried cells are used in for example tablets like BioGaia Dental^{™}. Products with live cells have to be consumed within 1-2 weeks since the bacteria die continuously. Freeze-dried bacteria can be stored for several years with maintained viability. However, freeze-dried bacteria are sensitive to humidity. One way of protecting the bacterial cells is to store them in oil. The freeze dried bacterial cells can be mixed directly with a suitable oil, or alternately the bacterial cell solution can be mixed with an oil and freeze dried together, leaving the bacterial cells completely immersed in oil. Suitable oils may be edible oils such as Olive oil in Extra Virgin, Virgin and other cold-pressed forms, Rapeseed oil which is prepared conventionally or cold-pressed, sunflower oil, soy oil, maize oil, cotton-seed oil, peanut oil, sesame oil, cereal germ oil such as wheat germ oil, grape kernel oil, palm oil and palm kernel oil, linseed oil. The viability of freeze-dried bacteria in oil is maintained for at least nine months. Optionally live cells can be added to one of the above oils and stored.

The compounds of the invention may be administered alone or in combination with pharmaceutically acceptable carriers or diluents, and such administration may be carried out in single or multiple doses.

Compositions may, for example, be in the form of tablets, resolvable tablets, capsules, pills sachets, vials, hard or soft capsules, aqueous or oily suspensions, aqueous or oily solutions, emulsions, powders, granules, syrups, elixirs, lozenges, reconstitutable powders, liquid preparations, creams, troches, hard candies, sprays, chewing-gums, creams, salves, jellies, gels, pastes, toothpastes, rinses, dental floss and tooth-picks, liquid aerosols, dry powder formulations, HFA aerosols or organic or inorganic acid addition salts.

The compositions of the invention may be in a form suitable for oral, topical, buccal administration.

Depending upon the disorder and patient to be treated and the route of administration, the compositions may be administered at varying doses.

### Salts/hydrates/solvates

The compounds of the present invention may be able to form salts with pharmaceutically acceptable acids or bases.

Suitable base addition salts of the compounds of the present invention include those formed with pharmaceutically acceptable salts such as alkali metal salts (for example lithium, sodium or potassium) alkaline earth metal salts (for example calcium or magnesium), organic amine salts (for example ammonium, triethylamine, morpholine, N-methylpiperidine, N-ethylpiperidine, procaine, dibenzylamine, N,N-dibenzylethylamine, hydroxyalkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine,N-benzyl-N-phenethylamine, dehydroabietylamine, N,N'-bisdehydroabietylamine, glucamine, N-methylglucamine), bases of the pyridine type (such as pyridine, collidine, quinine or quinoline) or amino acids (for example lysine).

It is also to be understood that compounds of the present invention can exist in solvated as well as unsolvated forms such as, e.g., hydrated forms.

### Oral/buccal

For oral or buccal administration, the compounds of the present invention may be combined with various excipients. Solid pharmaceutical preparations for oral administration often include binding agents (for example syrups, acacia, gelatin, tragacanth, polyvinylpyrrolidone, sodium lauryl sulphate, pregelatinized maize starch, hydroxypropyl methylcellulose, starches, modified starches, gum acacia, gum tragacanth, guar gum, pectin, wax binders, microcrystalline cellulose, methylcellulose, carboxymethylcellulose, hydroxypropyl methylcellulose, hydroxyethyl cellulose, hydroxypropyl cellulose, copolyvidone and sodium alginate), disintegrants (such as starch and preferably corn, potato or tapioca starch, alginic acid and certain complex silicates, polyvinylpyrrolidone, gelatin, acacia, sodium starch glycollate, microcrystalline cellulose, crosscarmellose sodium, crospovidone, hydroxypropyl methylcellulose and hydroxypropyl cellulose), lubricating agents (such as magnesium stearate, sodium lauryl sulfate, talc, silica polyethylene glycol waxes, stearic acid, palmitic acid, calcium stearate, camuba wax, hydrogenated vegetable oils, mineral oils, polyethylene glycols and sodium stearyl fumarate) and fillers (including high molecular weight polyethylene glycols. lactose, calcium phosphate, glycine magnesium stearate, starch, rice flour, chalk, gelatin, microcrystalline cellulose, calcium sulphate, and lactitol). Such preparations may also include preservative agents and anti-oxidants.

Liquid compositions for oral administration may be in the form of, for example, emulsions, syrups, or elixirs, or may be presented as a dry product for reconstitution with water or other suitable vehicle before use. Such liquid compositions may contain conventional additives such as suspending agents (e.g. syrup, methyl cellulose, hydrogenated edible fats, gelatin, hydroxyalkylcelluloses, carboxymethylcellulose, aluminium stearate gel, hydrogenated edible fats) emulsifying agents (e.g. lecithin, sorbitan monooleate, or acacia), aqueous or non-aqueous vehicles (including, edible oils, e.g. almond oil, fractionated coconut oil) oily esters (for example esters of glycerine, propylene glycol, polyethylene glycol or ethyl alcohol), glycerine, water or normal saline; preservatives (e.g. methyl or propyl p-hydroxybenzoate or sorbic acid) and conventional flavouring, preservative, sweetening or colouring agents. Diluents such as water, ethanol, propylene glycol, glycerin and combinations thereof may also be included.

Other suitable fillers, binders, disintegrants, lubricants and additional excipients are well known to a person skilled in the art.

### Controlled/delayed/prolonged release formulation

The compounds of the invention may also be administered in a controlled release formulation. The compounds are released at the required rate to maintain constant pharmacological activity for a desirable period of time. Such dosage forms provide a supply of a drug to the body during a predetermined period of time and thus maintain drug levels in the therapeutic range for longer periods of time than conventional non-controlled formulations. The compounds may also be formulated in controlled release formulations in which release of the active compound is targeted. For example, release of the compound may be limited to a specific region of the digestive system through the pH sensitivity of the formulation. Such formulations are well known to persons skilled in the art.

### Liposomes

The active compounds may be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

## Claims

1. An oral composition comprising:
a) one or more isolated weak or non acid-producing bacterial strain chosen from the group of early colonizing oral bacteria normally present in a healthy oral microflora, said group consisting of *Streptococcus oralis, Streptococcus vestibularis, Streptococcus sanguis, Streptococcus mitis, Eubacterium, Neisseria, Veillonella*; and
b) one or more substances having a pH-rising and/or pH-buffering capacity capable to maintain a pH above pH 5.5 up to pH 7.8, more preferable a pH above pH 6 to 7.5 and most preferably a pH above pH 6.5 to 7.2 in the oral cavity; and
c) components of the plant stevia.

2. An oral composition according to claim 1, wherein the weak or non acid-producing bacterial strain is *Streptococcus vestibularis.*

3. An oral composition according to claim 1, wherein the weak or non acid-producing bacterial strain is *Eubacterium saburreum* or *Eubacterium yurii*

4. An oral composition according to claim 1, wherein the weak or non acid-producing bacterial strain is *Neisseria mucosa* or *Neisseria subflava.*

5. An oral composition according to claim 1, wherein the weak or non acid-producing bacterial strain is *Veillonella parvula* or *Veillonella dispar.*

6. An oral composition according to claims 1-5, wherein the weak or non acid-producing bacterial strains are genetically modified.

7. An oral composition according to claim 1, wherein the pH-rising and/or pH-buffering substance is one, or a combination of two or more of the substances chosen from the group comprising bicarbonates, carbamides, phosphates, proteins and/or salts.

8. An oral composition according to claim 1, wherein the pH-rising and/or pH-buffering substances are used in a concentration ranging from 15-300 mmol/l oral composition, more preferably 30-200 mmol/l oral composition and most preferably 50-100 mmol/l oral composition.

9. An oral composition according to one or more of the preceding claims, wherein the bacteria are freeze dried.

10. An oral composition according to claim 9, wherein the freeze dried or living bacteria are immersed in oil.

11. An oral composition according to one or more of the preceding claims, wherein a lubricant has been added chosen from the group comprising edible oils, essential oils, glycerin, carboxymethyl cellulose, xanthan gum or animal mucin.

12. An oral composition according to one or more of the preceding claims, to which a fluoride compound from the group comprising sodium fluoride, monofluorophosphate or stannous fluoride has been added.

13. An oral composition according to one or more of the preceding claims, to which zinc and/or chlorine dioxide has been added.

14. An oral composition according to one or more of the preceding claims, to which an anti-inflammatory substance from the group comprising cortison, benzydamin, non-steroid anti-inflammatory drugs or herbal extracts such as for example calendula extract or tee tree oil, have been added.

15. An oral composition according to one or more of the preceding claims, to which an analgesic from the group comprising lidocaine or prilocaine has been added.

16. An oral composition according to one or more of the preceding claims, to which an anti-mycotic agent from the group comprising amfotericin, flucanozol or nystatin has been added.

17. An oral composition according to one or more of the preceding claims, to which one or more flavouring substances from the group comprising mints, fruit juices, liquorice, Stevia rebaudiana, steviosides, rebaudioside A, essential oils like eucalyptus oil, menthol has been added.

18. An oral composition according to one or more of the preceding claims 1-17, for use as a medicament.

19. Use of a composition according to one or more of the preceding claims 1-17 for the manufacture of a medicament for the re-establishment of good oral health.

20. An oral composition according to one or more of the preceding claims 1-17 for use in the re-establishment of good oral health.

21. An oral composition according to claim 20 for use in the re-establishment of good oral health in subjects suffering from oral dryness.

## Patentansprüche

1. Orale Zusammensetzung, umfassend:
a) einen oder mehrere isolierte (n) schwach- oder nicht-säureproduzierende(n) Bakterienstamm/-stämme, ausgewählt aus der Gruppe von frühkolonisierenden Mundbakterien, welche normalerweise in einer gesunden Mund-Mikroflora vorhanden sind, wobei die genannte Gruppe aus *Streptococcus oralis, Streptococcus vestibularis, Streptococcus sanguis, Streptococcus mitis, Eubacterium, Neisseria, veillonella* besteht; und
b) ein oder mehrere Substanzen mit der Fähigkeit, den pH-Wert zu erhöhen und/oder zu puffern, um einen pH-Wert über pH 5,5 bis zu pH 7,8, mehr zu bevorzugen einen pH-Wert über pH 6 bis 7,5, und am meisten zu bevorzugen einen pH-Wert über pH 6,5 bis 7,2 in der Mundhöhle aufrecht zu erhalten; und
c) Bestandteile der Pflanze Stevia.

2. Orale Zusammensetzung gemäß Anspruch 1, wobei der schwach- oder nicht-säureproduzierende Bakterienstamm *Streptococcus vestibularis* ist.

3. Orale Zusammensetzung gemäß Anspruch 1, wobei der schwach- oder nicht-säureproduzierende Bakterienstamm *Eubacterium saburreum* oder *Eubacterium yurii* ist.

4. Orale Zusammensetzung gemäß Anspruch 1, wobei der schwach- oder nicht-säureproduzierende Bakterienstamm *Neisseria mucosa* oder *Neisseria subflava* ist.

5. Orale Zusammensetzung gemäß Anspruch 1, wobei der schwach- oder nicht-säureproduzierende Bakterienstamm *veillonella parvula* oder *veillonella dispar* ist.

6. Orale Zusammensetzung gemäß den Ansprüchen 1 bis 5, wobei die schwach- oder nicht-säureproduzierenden Bakterienstämme genetisch verändert sind.

7. Orale Zusammensetzung gemäß Anspruch 1, wobei die Substanz, welche den pH-Wert erhöht und/oder puffert, eine Substanz, oder eine Kombination aus zwei oder mehrerer der Substanzen, ausgewählt aus der Gruppe umfassend Hydrogencarbonate, Carbamide, Phosphate, Proteine und/oder Salze ist.

8. Orale Zusammensetzung gemäß Anspruch 1, wobei die Substanzen, welche den pH-Wert erhöhen oder puffern, in einer Konzentration verwendet werden, die von 15 bis 300 mmol/l oraler Zusammensetzung, mehr zu bevorzugen 30 bis 200 mmol/l oraler Zusammensetzung, und am meisten zu bevorzugen 50 bis 100 mmol/l oraler Zusammensetzung reicht.

9. Orale Zusammensetzung gemäß einem oder mehreren der vorangehenden Ansprüche, wobei die Bakterien gefriergetrocknet werden.

10. Orale Zusammensetzung gemäß Anspruch 9, wobei die gefriergetrockneten oder lebenden Bakterien in Öl getaucht sind.

11. Orale Zusammensetzung gemäß einem oder mehreren der vorangehenden Ansprüche, wobei ein Schmiermittel, ausgewählt aus der Gruppe umfassend essbare Öle, essentielle Öle, Glycerin, Carboxymethylcellulose, Xanthangummi oder tierisches Mucin, zugegeben worden ist.

12. Orale Zusammensetzung gemäß einem oder mehreren der vorangehenden Ansprüche, zu der eine Fluoridverbindung, ausgewählt aus der Gruppe umfassend Natriumfluorid, Monofluorphosphat oder Zinn(II)-fluorid zugegeben worden ist.

13. Orale Zusammensetzung gemäß einem oder mehreren der vorangehenden Ansprüche, zu der Zink und/oder Chlordioxid zugegeben worden ist.

14. Orale Zusammensetzung gemäß einem oder mehreren der vorangehenden Ansprüche, zu der ein entzündungshemmender Stoff aus der Gruppe umfassend Cortison, Benzydamin, nichtsteroidaler Antirheumatika, oder Kräuterextrakte, wie zum Beispiel Calendulaextrakt oder Teebaumöl, zugegeben worden ist.

15. Orale Zusammensetzung gemäß einem oder mehreren der vorangehenden Ansprüche, zu der ein Analgetikum aus der Gruppe umfassend Lidocain oder Prilocain, zugegeben worden ist.

16. Orale Zusammensetzung gemäß einem oder mehreren der vorangehenden Ansprüche, zu der ein Antimykotikum aus der Gruppe umfassend Amfotericin, Flucanozol oder Nystatin, zugegeben worden ist.

17. Orale Zusammensetzung gemäß einem oder mehrerer der vorangehenden Ansprüche, zu der ein oder mehrere Aromastoff(e) aus der Gruppe umfassend Minzen, Obstsäfte, Lakritz, Stevia rebaudiana, Stevioside, Rebaudiosid A, essentielle Öle wie Eukalyptusöl, Menthol, hinzugefügt worden ist/sind.

18. Orale Zusammensetzung gemäß einem oder mehreren der vorangehenden Ansprüche 1 bis 17 zur Verwendung als Medikament.

19. Verwendung einer Zusammensetzung gemäß einem oder mehreren der vorangehenden Ansprüche 1 bis 17 zur Herstellung eines Medikaments zur Wiederherstellung guter Mundgesundheit.

20. Orale Zusammensetzung gemäß einem oder mehreren der vorangehenden Ansprüche 1 bis 17 zur Verwendung bei der Wiederherstellung guter Mundgesundheit.

21. Orale Zusammensetzung gemäß Anspruch 20 zur Verwendung bei der Wiederherstellung guter Mundgesundheit bei Subjekten, welche an Mundtrockenheit leiden.

## Revendications

1. Composition buccale comprenant :
a) une ou plusieurs souches bactériennes isolées, faiblement productrices ou non-productrices d'acide, choisies dans un ensemble de bactéries buccales colonisatrices précoces qui sont normalement présentes dans la flore microbienne buccale d'une personne en bonne santé, lequel ensemble est constitué de *Streptococcus oralis, Streptococcus vestibularis, Streptococcus sanguis, Streptococcus mitis, Eubacterium, Neisseria, Veillonella ;*
b) une ou plusieurs substances dotées du pouvoir d'élever le pH et/ou de le tamponner, capables de maintenir dans la cavité buccale un pH valant de plus de 5,5 à 7,8, de préférence un pH valant de plus de 6 à 7,5, et mieux encore un pH valant de plus de 6,5 à 7,2 ;
c) et des composants issus de la plante Stevia.

2. Composition buccale conforme à la revendication 1, dans laquelle la souche bactérienne faiblement productrice ou non-productrice d'acide est *Streptococcus vestibularis.*

3. Composition buccale conforme à la revendication 1, dans laquelle la souche bactérienne faiblement productrice ou non-productrice d'acide est *Eubacterium saburreum* ou *Eubacterium yurii*.

4. Composition buccale conforme à la revendication 1, dans laquelle la souche bactérienne faiblement productrice ou non-productrice d'acide est *Neisseria mucosa* ou *Neisseria subflava.*

5. Composition buccale conforme à la revendication 1, dans laquelle la souche bactérienne faiblement productrice ou non-productrice d'acide est *Veillonella parvula* ou *Veillonella dispar.*

6. Composition buccale conforme à l'une des revendications 1 à 5, dans laquelle les souches bactériennes faiblement productrices ou non-productrices d'acide sont des souches génétiquement modifiées.

7. Composition buccale conforme à la revendication 1, dans laquelle la substance élevant et/ou tamponnant le pH est une seule substance, ou une combinaison de deux substances ou plus, choisie(s) dans l'ensemble constitué par les bicarbonates, carbamides, phosphates, protéines et/ou sels.

8. Composition buccale conforme à la revendication 1, dans laquelle les substances élevant et/ou tamponnant le pH sont utilisées en une concentration valant de 15 à 300 millimoles par litre de composition buccale, de préférence de 30 à 200 millimoles par litre de composition buccale, et mieux encore de 50 à 100 millimoles par litre de composition buccale.

9. Composition buccale conforme à l'une ou plusieurs des revendications précédentes, dans laquelle les bactéries sont lyophilisées.

10. Composition buccale conforme à la revendication 9, dans laquelle les bactéries, lyophilisées ou vivantes, sont immergées dans une huile.

11. Composition buccale conforme à l'une ou plusieurs des revendications précédentes, à laquelle a été ajouté un lubrifiant choisi dans l'ensemble comprenant les huiles comestibles, huiles essentielles, glycérine, carboxyméthyl-cellulose, gomme xanthane et mucine animale.

12. Composition buccale conforme à l'une ou plusieurs des revendications précédentes, à laquelle a été ajouté un composé fluoré choisi parmi le fluorure de sodium, un monofluorophosphate, et le fluorure stanneux.

13. Composition buccale conforme à l'une ou plusieurs des revendications précédentes, à laquelle a ou ont été ajouté(s) du zinc et/ou du dioxyde de chlore.

14. Composition buccale conforme à l'une ou plusieurs des revendications précédentes, à laquelle a été ajoutée une substance anti-inflammatoire choisie dans l'ensemble constitué par la cortisone, la benzydamine, les médicaments anti-inflammatoires non-stéroïdiens et les extraits de végétaux comme l'extrait de calendula ou l'huile essentielle d'arbre à thé.

15. Composition buccale conforme à l'une ou plusieurs des revendications précédentes, à laquelle a été ajouté un analgésique choisi parmi les lidocaïne et prilocaïne.

16. Composition buccale conforme à l'une ou plusieurs des revendications précédentes, à laquelle a été ajouté un antimycotique choisi parmi les amphotéricine, fluconazole et nystatine.

17. Composition buccale conforme à l'une ou plusieurs des revendications précédentes, à laquelle a ou ont été ajoutée(s) une ou plusieurs substance(s) aromatisante(s) choisie(s) dans l'ensemble comprenant les menthes, jus de fruits, réglisse, Stevia rebaudiana, stéviosides, rébaudioside A, huiles essentielles comme l'essence d'eucalyptus, et menthol.

18. Composition buccale conforme à l'une ou plusieurs des revendications précédentes 1 à 17, pour utilisation en tant que médicament.

19. Utilisation d'une composition buccale, conforme à l'une ou plusieurs des revendications précédentes 1 à 17, pour la fabrication d'un médicament conçu pour rétablir une bonne santé buccale.

20. Composition buccale conforme à l'une ou plusieurs des revendications précédentes 1 à 17, pour utilisation afin de rétablir une bonne santé buccale.

21. Composition buccale conforme à la revendication 20, pour utilisation afin de rétablir une bonne santé buccale chez des sujets souffrant de sécheresse buccale.
